# EUROPÄISCHE PATENTSCHRIFT

(11) **EP 2 029 516 B1**
(45) Veröffentlichungstag und Bekanntmachung des Hinweises auf die Patenterteilung: **05.08.2015**
(21) Anmeldenummer: 07725655.0
(22) Anmeldetag: 30.05.2007
(51) Int. Cl.: C07C 67/32, C07C 69/63, C07C 68/02, C07C 69/96

(54) **VERFAHREN ZUR HERSTELLUNG VON FLUORIERTEN MOLEKÜLEN**
PROCESS FOR PREPARING FLUORINATED MOLECULES
PROCÉDÉ DE FABRICATION DE MOLÉCULES FLUORÉES

(30) Priorität: 08.06.2006 DE 102006027089
(43) Veröffentlichungstag der Anmeldung: 04.03.2009
(73) Patentinhaber: Bayer Intellectual Property GmbH, 40789 Monheim am Rhein (DE)
(72) Erfinder: GELLER, Thomas, 51519 Odenthal (DE); LUI, Norbert, 51519 Odenthal (DE)
(74) Vertreter: BIP Patents
(86) Internationale Anmeldenummer: PCT/EP2007/004764
(87) Internationale Veröffentlichungsnummer: WO 2007/140908

(56) Entgegenhaltungen:
- EP-A2- 0 163 435
- WO-A-2006/037887
- DE-A1- 1 493 194
- DE-A1- 2 542 251
- YAGUPOL'SKII, L. M. ET AL: "Perfluoroalkoxyacetic acids and their derivatives" UKRAINSKII KHIMICHESKII ZHURNAL (RUSSIAN EDITION) , 44(10), 1057-9 CODEN: UKZHAU; ISSN: 0041-6045, 1978, XP008083280
- FLOSSER D A ET AL: "A useful conversion of alcohols to alkyl fluorides" TETRAHEDRON LETTERS, ELSEVIER, AMSTERDAM, NL, Bd. 43, Nr. 23, 3. Juni 2002 (2002-06-03), Seiten 4275-4279, XP004354692 ISSN: 0040-4039 in der Anmeldung erwähnt

## Beschreibung

Die Erfindung betrifft das technische Gebiet der chemischen Verfahren zur Herstellung von fluorhaltigen Verbindungen, spezieller der Verfahren zur Herstellung von Carbonsäureestern, welche in α-Position (alpha) ein Fluoratom als Substituenten enthalten.

Es ist bereits bekannt, dass Ester, welche in α-Position (alpha) ein Fluoratom als Substituenten enthalten, durch die Umsetzung von α-Hydroxyestern mit Thionylchlorid zum Chlorsulfit, Umsetzung des Chlorsulfits mit einer Fluoridquelle zum Fluorsulfit und anschließende thermische Zersetzung, gegebenenfalls unter Amin oder Pyridinkatalyse, hergestellt werden können (DE 1122505), siehe Schema 1:

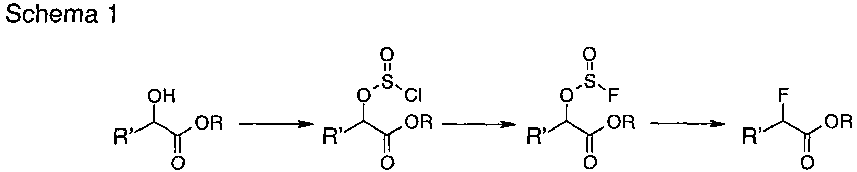

Durch den Einsatz von chiralen, nicht-racemischen α-Hydroxyestern kann diese Umsetzung bei ansonsten gleicher Reaktionsführung auch verwendet werden, um die Zielprodukte als chirale, nicht-racemische Verbindungen herzustellen (WO-A-2006/037887, FR-A1-2876100). Die nach diesen Verfahren benötigten Chlorsulfite der α-Hydroxyester können nach bekannten Verfahren hergestellt werden (z.B. EP-A-0056981, DE-A1-3102516).

Nachteilig bei dieser Art der Herstellung ist die Bildung von SO₂ als Abgas und die als Nebenreaktion beobachtete Zersetzung der Chlorsulfite zu den entsprechenden in α-Position chlorierten Estern (z.B. DE-A-3102516, dort als Hauptreaktion genutzt).

Die chlorierten Ester sind aufgrund sehr ähnlicher Eigenschaften nur schwer vom Zielprodukt abtrennbar.

Es ist ebenfalls bekannt, dass sich in α-Position fluorierte Ester auch durch Umsetzung von α-Hydroxyestern zu den entsprechenden Sulfonsäureestern und anschließende Umsetzung des Sulfonsäureesters mit einer Fluoridquelle (z.B. Kaliumfluorid) herstellen lassen (z.B. DE-A-4131242). Diese Reaktion kann enantioselektiv zur Herstellung chiraler, nicht-racemischer Zielprodukte genutzt werden, wenn man das Edukt in chiraler, nicht-racemischer Form einsetzt (siehe Schema 2 an Beispiel eines enantiomeren in α-Position fluorierten Esters).

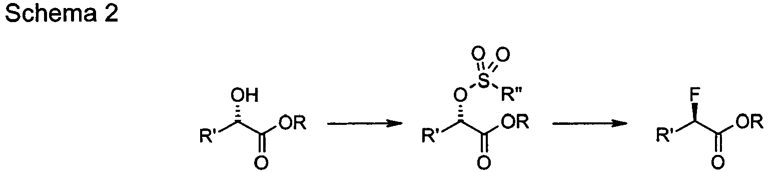

Bedingt durch das hohe Molekulargewicht der Abgangsgruppe wird es notwendig, während des Verfahrens mit relativ großen Massen umzugehen, um am Ende ein relativ leichtes Zielprodukt zu erhalten. Hierdurch und durch die schlechte biologische Abbaubarkeit der Abgangsgruppen kommt es im technischen Maßstab zu Problemen.

Aus DE-A-3836855 ist bekannt, dass α-fluorierte Ester durch Desaminierung einer Aminosäure mit Natriumnitrit in Gegenwart von Fluorwasserstoff und Pyridin erhalten werden können. Weiterhin ist in DE-A-3836855 die Ringöffnung eines Epoxids mit einer Mischung von HF und Pyridin beschrieben, welche nach Oxidation und Veresterung ebenfalls zu in α-Position fluorierten Estern führt. Bei beiden Verfahren sind die verwendeten Mengen an Fluorwasserstoff und Pyridin relativ hoch, welches deren technische Einsetzbarkeit erschwert. Darüber hinaus führt die Desaminierung im Fall der Herstellung von chiralen, nicht-racemischen Produkten nur zu unbefriedigenden Enantiomerenüberschüssen (weniger als 60 %).

Desweiteren ist aus Tetrahedron Lett., 2002, 43, 4275 - 4279 bekannt, dass Alkylfluorformiate in Gegenwart von Hexabutylguanidiniumfluorid thermisch zu Fluoralkanen zersetzt werden können (siehe Schema 3), wobei die Reaktion weitgehend enantioselektiv verläuft. Die dabei eingesetzten Alkylfluorformiate lassen sich nach J. Org. Chem. 1956, 21, 1319-1320 bzw. Tetrahedron Lett., 2002, 43, 4275 - 4279 durch Umsetzung von Hydroxyalkanen mit Fluorbromphosgen, Fluorchlorphosgen, Difluorphosgen bzw. Kaliumfluorid + UV-Strahlung herstellen.

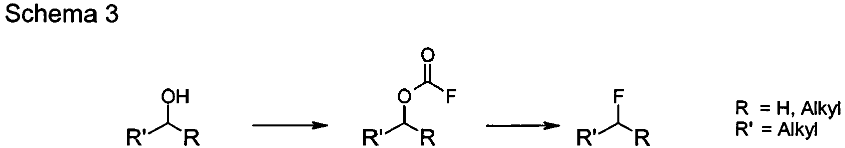

Da in α-Position fluorierte Ester, insbesondere in ihrer chiralen, nicht-racemischen Form, wichtige Intermediate für biologisch aktive Verbindungen darstellen, ist es wünschenswert einen Herstellungsweg zu finden, der die fluorierten Ester in einfacher Weise technisch darstellbar macht.

Gegenstand der Erfindung ist ein Verfahren zur Herstellung von Verbindungen der Formel (IV), gegebenenfalls in optisch aktiver Form, 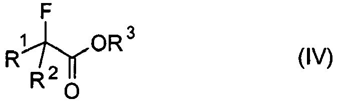 worin
- R¹: für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Aryl, Heterocyclyl, Phenyl-(C₁-C₆)-alkyl oder Heterocyclyl-(C₁-C₆)-alkyl, wobei jeder der letztgenannten 4 Reste am Ring am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
- R²: für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder gegebenenfalls substituiertes Aryl, vorzugsweise Phenyl, das unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist, steht,
- R³: für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
- R⁴: für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
dadurch gekennzeichnet, dass man
(a) eine Verbindung der Formel (I) 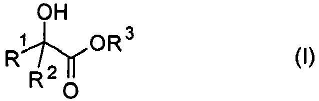 worin R¹, R² und R³ wie in Formel (IV) definiert sind, mit einer Dihalogencarbonylverbindung oder einem Äquivalent davon zur Verbindung der Formel (II) [Variante (a1)] oder (III) [Variante (a2)] umsetzt, 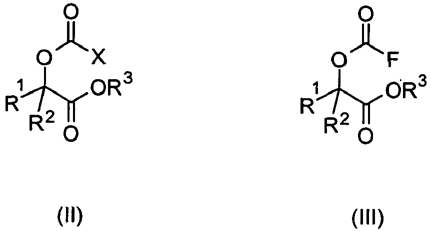 wobei in den Formeln (II) und (III)
   R¹, R² und R³ wie in Formel (IV) definiert sind und
   X ein Halogenatom aus der Gruppe Cl oder Br bedeutet,
   und
(b) für den Fall, dass in Stufe (a) nach Variante (a1) die Verbindung (II) erhalten worden ist, die Verbindung (II) mit einem Fluorierungsreagenz zur Verbindung der genannten Formel (III) umsetzt,
(c) die nach Stufe (a) oder (b) erhaltene Verbindung der Formel (III) thermisch, gegebenenfalls in Gegenwart eines Katalysators, unter Decarboxylierung zur Verbindung der genannten Formel (IV) umsetzt.

In der Formel (I) und allen nachfolgenden Formeln können die Reste Alkyl auch in den zusammengesetzten Bedeutungen wie Alkoxy, Haloalkyl und Haloalkoxy sowie die entsprechenden ungesättigten und/oder substituierten Reste im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl or tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl. Im Falle von substituiertem Cycloalkyl werden cyclische Systeme mit Substituenten umfaßt, wobei die Substituenten mit einer Doppelbindung am Cycloalkylrest, z. B. eine Alkylidengruppe wie Methyliden, gebunden sind. Im Falle von substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl.

Halogen bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält. Vorzugsweise ist er ein heteroaromatischer Ring mit einem Heteroatom aus der Gruppe N, O und S, beispielsweise Pyridyl, Pyrrolyl, Thienyl oder Furyl; weiterhin bevorzugt ist er ein entsprechender heteroaromatischer Ring mit 2 oder 3 Heteroatomen, z. B. Pyrimidinyl, Pyridazinyl, Pyrazinyl, Triazinyl, Thiazolyl, Thiadiazolyl, Oxazolyl, Isoxazolyl, Pyrazolyl, Imidazolyl und Triazolyl. Weiterhin bevorzugt ist er ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thienyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Aryl-, Phenyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugt Substituenten für die Subtituentenebenen sind beispielsweise:
Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Die Formel (I) und nachfolgende Formeln umfassen auch alle Stereoisomeren und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können in den bevorzugten enantioselektiven Verfahrensweise gezielt hergestellt werden, wenn optisch aktive Ausgangsstoffe eingesetzt werden.

Bevorzugte Kettenlängen für Alkyl, Alkenyl, Alkinyl in den Resten R¹, R², R³ und R⁴ sind C₁-C₁₂, besonders bevorzugt C₁-C₆, ganz besonders C₁-C₄,

Bevorzugte Ringröße für Cycloalkyl in den R¹, R², R³ und R⁴ ist C₃-C₇, insbesondere C₃-C₆.

Vorzugsweise steht
- R¹: für Wasserstoff, (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl, Heterocyclyl mit 3 bis 6 Ringatomen, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet.

Insbesondere steht
- R¹: für Wasserstoff, (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl.

Ganz besonders steht R¹ für Wasserstoff oder (C₁-C₄)-Alkyl.

Bevorzugt steht
- R²: für Wasserstoff, (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder gegebenenfalls substituiertes Aryl, vorzugsweise Phenyl, das unsubstituiert oder durch einen oder mehrere Reste aus der Gruppe Halogen, (C₁-C₆)-Alkyl, (C₁-C₆)-Alkoxy, (C₁-C₆)-Haloalkyl und (C₁-C₆)-Haloalkoxy substituiert ist.

Insbesondere steht
- R²: für Wasserstoff, (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl.

Ganz besonders steht R² für Wasserstoff oder (C₁-C₄)-Alkyl.

Bevorzugt steht
- R³: für (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl, Phenyl, Heterocyclyl, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet,

Insbesondere steht
- R³: für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl.

Ganz besonders steht R³ für (C₁-C₄)-Alkyl.

Bevorzugt steht
- R⁴: für (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl, Phenyl, Heterocyclyl, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet.

Insbesondere steht
- R⁴: für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl.

Ganz besonders steht R⁴ für (C₁-C₄)-Alkyl.

Bevorzugt sind auch Verfahren mit Verbindungen, worin zwei oder mehrere der obengenannten bevorzugten Merkmale kombiniert werden.

Besonders bevorzugt sind dabei die Verfahren mit enantioselektiven Stufen bis zur Herstellung von optisch aktiven Verbindungen (IV).

In der bevorzugten Ausführungsform werden die fluorierten Ester schnell und ohne ökologisch schwierige Nebenkomponenten herstellbar. Die eingesetzten Reagenzien führen durch ihr relativ geringes Molekulargewicht zu niedrigen Masseströmen. Das erfindungsgemäße Verfahren stellt somit eine Bereicherung des Standes der Technik dar, da es eine sehr vorteilhafte Herstellung von α-fluorierten Estern aus einfach zugänglichen α-Hydroxyestern erlaubt. Von den bekannten Verfahren aus war nicht zu erwarten, dass die erfindungsgemäße Herstellung von Verbindungen der Formel (IV) effizient und mit der hohen Ausbeute und Reinheit gelingen würde, insbesondere im Fall der Herstellung von chiralen nicht-racemischen Verbindungen. Dies gilt auch wegen der Unterschiede in den Reaktivitäten, die bekanntermaßen zwischen Fluorverbindungen im Vergleich zu Chlor- und Bromverbindungen oftmals sehr groß sind.

Das Verfahren zur Herstellung der Verbindungen (IV) besteht nach einer Variante aus den drei Stufen (a1) + (b) + (c) (siehe Schema 4), nach einer anderen Variante aus den zwei Stufen (a2) + (c) (siehe Schema 5).

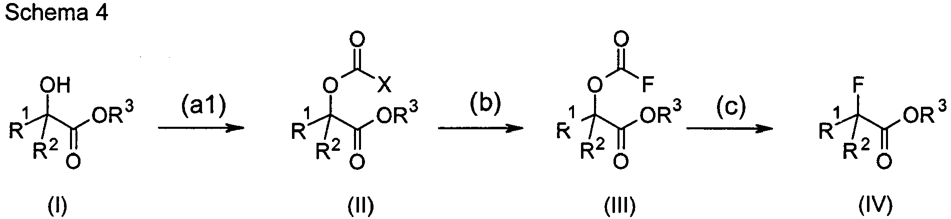

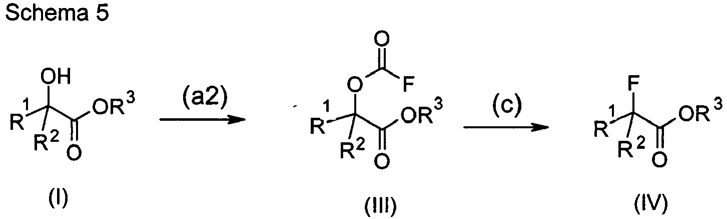

Gegenstand der Erfindung ist auch die Kombination der Verfahrensstufen (b) + (c) oder das Verfahren (c), wobei die eingesetzten Verbindungen (II) bzw. (III) dann auch auf anderem Wege hergestellt werden können.

Gegenstand der Erfindung sind auch die einzelnen Stufen (a2) und (b), die Verbindungen der Formel (III) und die Kombinationen (a1) + (b) und (a2) + (b).

### Zur Verfahrensstufe (a), Varianten (a1) und (a2):

Die Hydroxycarbonsäureester der Formel (I) sind weitgehend bekannt oder können analog bekannten Verfahren hergestellt werden (siehe z. B. EP-A-0163435 und dort zitierte Literatur).

Deren Umsetzung zu Chlorformiaten der Formel (II) (X = Cl) nach Variante (a1) ist bereits teilweise beschrieben (siehe auch EP-A-0163435). Die Chlor- oder Bromformiate der Formel (II) können durch Umsetzung der Verbindungen (I) mit einer Dihalogencarbonylverbindung oder einem Äquivalent davon hergestellt werden, wobei als Dihalogenverbindung oder dessen Äquivalent beispielsweise Phosgen (Cl-CO-Cl), Carbonyldibromid (Br-CO-Br), Carbonylbromidchlorid (Cl-CO; Br), Diphosgen, Triphosgen, etc. eingesetzt werden können.

Die Fluorformiate (III) gemäß einem der Ansprüche 11 und 12 und die Herstellung von Fluorformiaten (III) nach Variante (a2) sind neu. Im Unterschied zur Variante (a1) werden In Variante (a2) fluorhaltige Dihalogenverbindungen oder dessen Äquivalente eingesetzt, beispielsweise Carbonyldifluorid (F-CO-F), Carbonylfluoridchlorid (F-CO-Cl) Carbonylfluoridbromid (F-CO-Br), etc.

Die Variante (a2) kann ansonsten unter den Verfahrensbedingungen durchgeführt werden, wie sie analog für die Variante (a1) bekannt sind.

Bevorzugt sind auch die Ausführungen der Variante (a1) und (a2) mit Umsetzung von optisch aktiven Verbindungen der Formel (I) zu optisch aktiven Verbindungen der Formel (II) bzw. (III). Die Verfahrensstufe (a) kann somit enantioselektiv durchgeführt werden, was für die Herstellung von optisch aktiven Verbindungen (IV) nach dem Gesamtverfahren von Bedeutung und vorteilhaft ist.

### Zur Verfahrenstufe (b):

Die erfindungsgemäßen Umsetzung der Halogenformiate der Formel (II) mit einem Fluorierungsmittel wird in der Regel bei Temperaturen zwischen -15°C und 150°C, vorzugsweise zwischen -10°C und 100°C, besonders bevorzugt zwischen 0°C und 50°C durchgeführt.

Als Fluorierungsmittel können übliche Fluorierungsreagenzien, vorzugsweise salzartige, fluorhaltige Verbindungen verwendet werden, beispielsweise Hydrogenfluorid (HF) oder Mischungen bzw. Salze von Hydrogenfluorid mit organischen Basen wie organische Aminbasen, beispielsweise Pyridin/Hydrogenfluorid, Triethylamin/HF oder Tributylamin/HF. Entsprechend gut geeignet sind Alkalimetallfluoride wie Natriumfluorid, Kaliumfluorid, Ammoniumfluorid, mit organischen Resten substituierte Ammonium- oder Phosphoniumfluoride, vorzugsweise quaternäre Ammonium- oder Phosphoniumfluoride, wie beispielsweise Tetrabutylammoniumfluorid oder Tetraphenylphosphoniumfluorid. Bevorzugt werden Pyridin/Hydrogenfluorid, Triethylamin/HF, NaF oder KF eingesetzt.

Zur Herstellung der Verbindungen der Fluorformiate der Formel (III) wird in Stufe (b) das Fluorierungsreagenz vorzugsweise äquimolar oder im Überschuss eingesetzt. Zweckmäßig werden in der Regel je Mol Edukt der Formel (II) zwischen 1.0 und 10 Moläquivalente, vorzugsweise zwischen 1,0 und 1,6 Moläquivalente Fluorierungsreagenz, insbesondere dabei ein stöichiometrischer Überschuss an Fluorierungsreagenz eingesetzt. Unter 1 Moläquivalent wird dabei ein Mol eines Fluorierungsreagenzes verstanden, das ein Mol Fluoratom pro Mol Reagenz überträgt. Entsprechend bedeutet 1 Moläquivalent auch ein halbes Mol eines Fluorierungsreagenzes, das pro Mol Reagenz zwei Fluoratome überträgt (z. B. ein Erdalkalifluorid).

Die Umsetzung in Stufe (b) kann mit oder ohne Lösungsmittel ausgeführt werden. Als Lösungsmittel bei der Umsetzung werden bevorzugt inerte Lösungsmittel eingesetzt z.B. alkylierte Aromaten, halogenierte Aromaten, halogenierte Alkane, N,N-dialkylierte Amide, alkylierte Pyrrolidone, Ether, Nitrile, Pyridine und Sulfolan. Besonders bevorzugt sind Chlorbenzol, Dichlorbenzol, Trichlorbenzol, Methylenchlorid, Chloroform, Sulfolan, Dimethylacetamid, Dimethylformamid, Acetonitril, Benzonitril und Pyridin. Ganz besonders bevorzugt sind Methylenchlorid, Pyridin, Sulfolan und Dimethylacetamid.

Die Verfahrensstufe (b) kann, wie auch alle übrigen erfindungsgemäßen Verfahren, zweckmäßig bei Normaldruck durchgeführt werden. Es ist jedoch auch möglich, unter erhöhtem oder vermindertem Druck - vorzugsweise zwischen 0,1 bar und 10 bar - zu arbeiten. Optional kann in Stufe (b) ein Katalysator zugesetzt werden, der speziell den Austausch des Halogens X = Cl oder Br in der Verbindung (II) gegen Fluor katalysiert und dabei vorzugsweise die nucleophile Reaktivität des Fluoridanions im Reaktionsmedium erhöht. Geeignete Katalysatoren sind beispielsweise Kronenether (z.B. 18-[K]-6), Polyethylenglycoldialkylether, quaternäre Ammoniumfluoride oder quaternäre Phosphoniumfluoride oder CsF.

### Zur Verfahrenstufe (c):

Die erfindungsgemäße Umsetzung (Decarboxylierung) der Fluorformiate der Formel (III) zu den α- fluorierten Estern (IV) wird in der Regel bei Temperaturen zwischen -15°C und 200°C, vorzugsweise zwischen 60°C und 200°C, besonders bevorzugt 90°C und 180°C durchgeführt.

Die Decarboxylierung kann ohne weitere Zusätze oder vorzugsweise in Gegenwart eines Decarboxylierungsreagenzes oder -katalysators, gegebenenfalls in Gegenwart einer Fluoridquelle wie beispielsweise Kaliumfluorid oder Hydrogenfluorid, (gemeinsam hier auch als "Katalysator" bezeichnet) erfolgen.

Als Decarboxylierungsreagenzien oder -katalysatoren können beispielsweise Alkalihalogenide (zum Beispiel KF, CsF, oder Mischungen aus diesen), aromatische oder heteroaromatische tertiäre Amine und -Pyridine, beispielsweise N,N-Dimethylaminopyridin, oder Phasen-Transfer-Katalysatoren oder Mischungen aus diesen verwendet werden.

Zu den Phasen-Transfer-Katalysatoren gehören beispielsweise:
(A) quartäre Phosphonium- oder Ammoniumverbindungen der Formel (V), 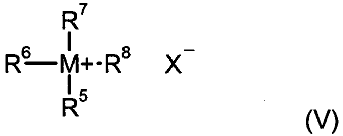 in welchen
   - R⁵, R⁶, R⁷ und R⁸: unabhängig voneinander für C₁-C₂₂-Alkyl, jeweils gegebenenfalls substituiertes Aryl oder (C₁-C₄-Alkyl)aryl steht, wobei Aryl die Bedeutung Phenyl oder Naphthyl hat und die besagten Substituenten Halogen, C₁-C₄-Alkyl, C₁-C₄-Alkoxy, Nitro oder Cyano bedeuten,
   - X⁻: ein Äquivalent eines nucleophilen Anions (z.B. Cl⁻, Br⁻, J⁻) bedeutet,
   - M⁺: für N oder P steht,
   oder
(B) Amidophosphoniumsalze der Formel (VI), 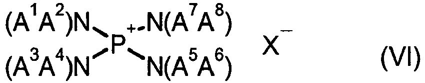 in welchen
   - A¹, A², A³, A⁴, A⁵, A⁶, A⁷ und A⁸: unabhängig voneinander für C₁-C₁₂-Alkyl oder C₂-C₁₂-Alkenyl, C₄-C₈-Cycloalkyl, C₆-C₁₂-Aryl, C₇-C₁₂-Aralkyl stehen, oder
   - A¹A², A³A⁴, A⁵A⁶ und A⁷A⁸: unabhängig voneinander direkt oder über O oder N-A⁹ miteinander zu einem 3- bis 7-gliedrigen Ring verbunden sind,
   - A⁹: für C₁-C₄-Alkyl steht,
   - X⁻: für ein nucleophiles Anion steht (z.B. Cl⁻, Br⁻, J⁻), oder
(C) Verbindungen der Formel (VII), 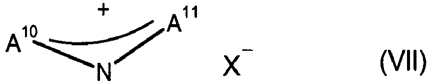 in welchen
   - A¹⁰ und A¹¹: unabhängig voneinander für einen der folgenden Reste stehen

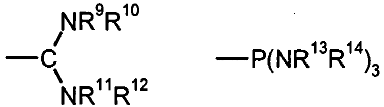

   - R⁹, R¹⁰, R¹¹, R¹², R¹³ und R¹⁴: unabhängig voneinander für C₁-C₁₀-Alkyl, C₂-C₁₀-Alkenyl oder C₆-C₁₂-Aryl stehen oder
   - R⁹R¹⁰, R¹¹R¹², R¹³R¹⁴: paarweise mit den jeweils verbundenen N-Atomen direkt miteinander zu einem 3- bis 5-gliedrigen, gesättigten oder ungesättigten Ring verbunden sind, der ein Stickstoffatom und ansonsten Kohlenstoffatome enthält wobei der Rest 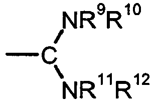 auch für einen gesättigten oder ungesättigten 4- bis 8-gliedrigen Ring stehen kann, der zwei Stickstoffatome und sonst Kohlenstoffatome enthält,
   - X⁻: ein Äquivalent eines nucleophilen Anions (z.B. Cl⁻, Br⁻, J⁻) bedeutet,
   oder
(D) Hexaalkylguanidiniumsalze der Formel (VIII), 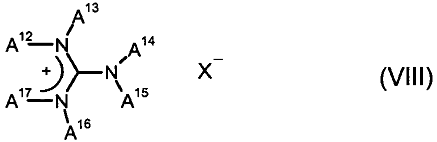 in welchen
   - A¹², A¹³, A¹⁴, A¹⁵, A¹⁶ und A¹⁷: unabhängig voneinander für C₁-C₁₂-Alkyl oder C₂-C₁₂-Alkenyl, C₄-C₈-Cycloalkyl, C₆-C₁₂-Aryl, C₇-C₁₂-Aralkyl stehen, oder
   - A¹²A¹³, A¹⁴A¹⁵ und A¹⁶A¹⁷: unabhängig voneinander direkt oder über O oder N-A¹⁸ miteinander zu einem 3- bis 7-gliedrigen Ring verbunden sind,
   - A¹⁸: für C₁-C₄-Alkyl steht,
   - X⁻: ein Äquivalent eines nucleophilen Anions bedeutet.

Bevorzugt werden N,N-Dimethylaminopyrimidin (DMAP), CsF, Tetraalkylammoniumsalze, Tetraarylphosphoniumsalze und die Hexaalkylguanidiniumsalze eingesetzt; besonders bevorzugt sind CsF, Tetraalkylammoniumchloride und -bromide.

Die genannten Verbindungen sind dem Fachmann als Phasen-Transfer-Katalysatoren bekannt.

Optional kann ein weiterer Katalysator, wie beispielsweise ein Kronenether (z.B. 18-[K]-6) oder ein Polyethylenglycoldialkylether zugesetzt werden.

Bevorzugt ist die enantioselektive Verfahrensweise der Stufe (c) unter Anwendung von Decarboxylierungsreagenzien oder -katalysatoren. Die Unterscheidung von Reagenzien und Katalysatoren ist nur sinnvoll hinsichtlich der unterschiedlichen Mengen, die hierbei optimal sind; beide Stoffe haben gemeinsam, dass sie die Decarboxylierung fördern ("katalysieren"). Geeignete Decarboxylierungsreagenzien oder -katalysatoren für die enantioselektive Verfahrensweise sind Alkalihalogenide, vorzugsweise Fluoride wie KF, CsF oder Mischungen von Fluoriden, oder aromatische oder heteroaromatische tertiäre Amine wie N-substituierte Aminopyridine, beispielsweise N,N-Dimethylaminopyridin, oder die genannten Phasen-Transfer-Katalysatoren oder Mischungen aus diesen.

Zur Herstellung der Verbindungen der Formel (IV) wird das Decarboxylierungsreagenz bzw. der Decarboxylierungskatalysator in der Regel in einem Verhältnis zwischen 0,005 Mol und 6 Mol, vorzugsweise zwischen 0,005 Mol und 2 Mol, besonders bevorzugt in einem Verhältnis zwischen 0,01 Mol und 2 Mol pro Mol Verbindung der Formel (III) eingesetzt.

Die Decarboxylierungsreaktion kann mit oder ohne Lösungsmittel ausgeführt werden. Als Lösungsmittel bei der Umsetzung werden bevorzugt Lösungsmittel aus der Gruppe der halogenierte Aromaten, halogenierte Alkane, N,N-dialkylierte Amide, N-alkylierte Pyrrolidone, Ether, Pyridine, Ester, Nitrile und Sulfolan eingesetzt. Besonders bevorzugt sind Chlorbenzol, Dichlorbenzol, Trichlorbenzol, Sulfolan und Dimethylacetamid. Ganz besonders bevorzugt sind Chlorbenzol, Sulfolan Produkte der Reaktion selbst und Dimethylacetamid.

Bevorzugt ist auch die Umsetzung ohne Lösungsmittel, wobei die Edukte und Produkte der Reaktion selbst als Lösungsvermittler oder Lösungsmittel dienen.

Alle erfindungsgemäßen Verfahren können zweckmäßig unter Normaldruck durchgeführt werden. Es ist jedoch auch möglich, unter erhöhtem oder vermindertem Druck - in der Regel zwischen 0,1 bar und 10 bar - zu arbeiten.

In den Folgenden Ausführungsbeispielen beziehen sich die Mengenangaben (inklusive Prozentangaben) auf das Gewicht, wenn nicht speziell Anderes definiert ist.

### Beispiele 1 bis 5

Je 1,0 g Methyl 2S-[(fluorcarbonyl)oxy]propanoat wurde nach den in der Tabelle 1 verzeichneten Bedingungen zum 2(R)-Fluorpropionsäuremethylester umgesetzt. Die aufgeführte Temperatur gibt die mittlere Badtemperatur des Heizmediums an. Die Ansätze wurden ohne Ausdestillieren des Produkts ausgeführt. Die Umsätze zum Zielprodukt sind in der Tabelle 1 verzeichnet.

Das Produkt 2(R)-Fluorpropionsäuremethylester wurde auch durch sein NMR-Spektrum charakterisiert:
¹H NMR (400 MHz, CD₃CN): δ = 1.51 (dd, 3H, H-C3, *J₁* = 23,9 Hz, *J₂* = 6,8 Hz), 3.73 (s, 3H, Ester-Methyl), 5,06 (dq, 1 H, 48,3 Hz, *J₂* = 6,9 Hz).

Der Enantiomerenüberschuss wurde durch Gaschromatographie an einer chiralen Trägerphase bestimmt.

**Tabelle 1**

| Nr. | Kat. | Kat. [eq] | KF [eq.] | T [°C] | Konz [%] | t [h] | Lsgm. | Umsatz [%] | ee* [%] |
|---|---|---|---|---|---|---|---|---|---|
| 1 | DMAP | 0,1 | / | 90 | 20 | 6 | MCB | 33 | 95 |
| 2 | DMAP | 0,1 | / | 90 | / | 6 | / | 48 | 88 |
| 3 | DMAP | 0,1 | / | 90 | 20 | 6 | Produkt | 83 | 95 |
| 4 | CsF | 0,1 | / | 160 | 20 | 6 | DMAA | 42 | 96 |
| 5 | 18[K]6 | 0,05 | 2 | 120 | 20 | 6 | DMAA | 63 | 73 |

| | | | | | | | | | |
|---|---|---|---|---|---|---|---|---|---|
| Abkürzungen zu Tabelle 1: ee = Enantiomerenüberschuss * = Enantiomerenüberschuß am Ende der Reaktionszeit; Kat. = Decarboxylierungskatalysator Kat [eq] = Menge Kat. in Moläquivalenten bezogen auf Edukt T = Reaktionstemperatur (mittlere Temperatur des Heizmediums) Konz = Konzentration an Edukt am Anfang der Reaktion Lsgm = Lösungsmittel Umsatz = Umsatz der Reaktion bezogen auf Edukt DMAP = N,N-Dimethylaminopyridin, MCB = Monochlorbenzol, DMAA = Dimethylacetamid, 18[K]6 = Kronenether 18-Krone-6 | | | | | | | | | |

### Beispiele 6 bis 10

Je 1,0 g racemisches Methyl 2-[(fluorcarbonyl)oxy]propanoat wurde nach den in der Tabelle 2 verzeichneten Bedingungen zum racemischen 2-Fluorpropionsäuremethylester umgesetzt. Die aufgeführte Temperatur gibt die mittlere Badtemperatur des Heizmediums an. Die Ansätze wurden ohne Ausdestillieren des Produkts ausgeführt. Die Umsätze zum Zielprodukt sind in der Tabelle 2 verzeichnet.

Das Produkt 2(*R*)-Fluorpropionsäuremethylester wurde auch durch sein NMR-Spektrum charakterisiert: ¹H NMR (400 MHz, CD₃CN): 8 = 1,51 (dd, 3H, H-C3, *J₁* = 23,9 Hz, *J₂* = 6,8 Hz), 3,73 (s, 3H, Ester-Methyl), 5,06 (dq, 1 H, 48,3 Hz, *J₂* = 6,9 Hz).

**Tabelle 2**

| Nr | Kat. | [eq] | KF [eq.] | T [°C] | Konz [%] | t [h] | Lsgm. | Umsatz |
|---|---|---|---|---|---|---|---|---|
| 6 | HBGCl | 0,1 | / | 90 | 40 | 6 | DMAA | 83 |
| 7 | HBGCl | 0,1 | / | rfx. | 40 | 6 | THF | 83 |
| 8 | Bu₄NBr | 0,1 | / | 90 | 20 | 6 | DMAA | 91 |
| 9 | Bu₄NBr | 0,1 | / | 90 | 20 | 6 | Xylol | 91 |
| 10 | Bu₄NCl | 0,1 | / | 90 | 20 | 6 | MCB | 83 |

| | | | | | | | | |
|---|---|---|---|---|---|---|---|---|
| Abkürzungen zu Tabelle 2: Siehe Abkürzungen zu Tabelle 1 Weitere Abkürzungen zu Tabelle 2: HBGCl = Hexabutylguanidiniumchlorid, Bu₄NBr = Tetrabutylammoniumbromid, Bu₄NCl = Tetrabutylammoniumchlorid, THF = Tetrahydrofuran | | | | | | | | |

### Beispiel 11

4,7 g Pyridin wurden in 10 ml Dichlormethan vorgelegt und bei Raumtemperatur mit 1,9 g Pyridin-HF-Komplex versetzt (M = 99,11 g/mol). In diese Mischung wurden 10 g Methyl 2-[(chlorcarbonyl)oxy]propanoat (ee. 99 %) zugetropft und die resultierende Reaktionsmischung über Nacht bei Raumtemperatur gerührt. Anschließend wurde die Mischung auf halbkonzentrierte Salzsäure geben, die organische Phase abgetrennt und die wässrige Phase mit Dichlormethan nachextrahiert. Die vereinigten organischen Phasen wurden getrocknet (Na₂SO₄) und eingeengt. Man erhielt 7,8 g Methyl 2-[(fluorcarbonyl)oxy]propanoat mit einem Gehalt von 89 % (Ausbeute: 77 %, ee: 99 %). Die Analysen des Enantiomerenüberschusses wurden mittels chiraler GC vorgenommen.
¹H NMR (400 MHz, CD₃CN): δ = 1,55 (dd, 3H, H-C3, *J₁* = 7,1 Hz, *J₂* = 1.6 Hz), 3,76 (s, 3H, Ester-Methyl), 5,12 (dq, 1H, *J₁* = 7,6 Hz, *J₂* = 1,1 Hz).

### Beispiel 12

11.2 g 2-*RS*-Ethylhexyl-2'*S*-[(chlorcarbonyl)oxy]propanoat wurden bei Raumtemperatur in eine Mischung aus 3.7 g Kaliumfluorid und 0.56 g des Kronenethers 18-Krone-6 in 25 g Methylenchlorid zugetropft. Nach Rühren über Nacht erhielt man 9.2 g 2-*RS*-Ethylhexyl-2'*S*-[(fluorcarbonyl)oxy]propanoat mit einem Gehalt von 82 % (Ausbeute: 72 % der Theorie).
¹³C NMR (151 MHz, CD₃CN): δ = 11.2 (CH₃CH₂-), 14.3 (C6), 16.9 (C3'), 23.6 (C5), 24.3, 24.4 (CH₃CH₂-), 29.5 (C4), 30.9 (C3), 39.5 (C2), 68.7 (C1), 76.2 (C2'), 145.5 (COF, J = 1.9 Hz), 169.9 (C1').

## Patentansprüche

1. Verfahren zur Herstellung von Verbindungen der Formel (IV), gegebenenfalls in optisch aktiver Form, 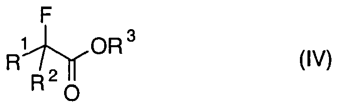 worin
R¹ für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis 12 bedeutet, oder Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R² für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis 12 bedeutet, oder gegebenenfalls substituiertes Aryl, vorzugsweise Phenyl, das unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist, steht,
R³ für (C₁-C₂₄)-Alkyl, (C₂-C₂₄)-Alkenyl, (C₂-C₂₄)-Alkinyl, (C₃-C₉)-Cycloalkyl, (C₁-C₆)-Alky)-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R⁴ für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
**dadurch gekennzeichnet, dass** man
(a) eine Verbindung der Formel (I) 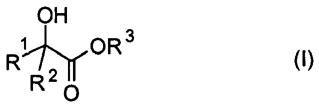 worin R¹, R² und R³ wie in Formel (IV) definiert sind,
mit einer Dihalogencarbonylverbindung oder einem Äquivalent davon zur Verbindung der Formel (II) [Variante (a1)] oder (III) [Variante (a2)] umsetzt, 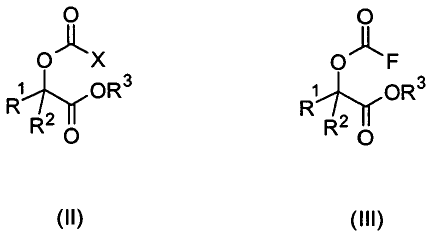 wobei in den Formeln (II) und (III)
R¹, R² und R³ wie in Formel (IV) definiert sind und
X ein Halogenatom aus der Gruppe Cl oder Br bedeutet,
und
(b) für den Fall, dass in Stufe (a) nach Variante (a1) die Verbindung (II) erhalten worden ist, die Verbindung (II) mit einem Fluorierungsreagenz zur Verbindung der genannten Formel (III) umsetzt,
(c) die nach Stufe (a) oder (b) erhaltene Verbindung der Formel (III) thermisch, gegebenenfalls in Gegenwart eines Katalysators, unter Decarboxylierung zur Verbindung der genannten Formel (IV) umsetzt.

2. Verfahren nach Anspruch 1, **dadurch gekennzeichnet, dass** in der Formel (IV)
R¹ für Wasserstoff, (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl, Heterocyclyl mit 3 bis 6 Ringatomen, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet, steht,
R² für Wasserstoff, (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder gegebenenfalls substituiertes Aryl, vorzugsweise Phenyl, das unsubstituiert oder durch einen oder mehrere Reste aus der Gruppe Halogen, (C₁-C₆)-Alkyl, (C₁-C₆)-Alkoxy, (C₁-C₆)-Haloalkyl und (C₁-C₆)-Haloalkoxy substituiert ist, steht,
R³ für (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl, Phenyl, Heterocyclyl, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet, steht, und
R⁴ für (C₁-C₁₂)-Alkyl, (C₂-C₁₂)-Alkenyl, (C₂-C₁₂)-Alkinyl, (C₃-C₆)-Cycloalkyl, (C₁-C₄)-Alkyl-(C₃-C₆)-cycloalkyl, Phenyl, Heterocyclyl, Phenyl-(C₁-C₄)-alkyl oder Heterocyclyl-(C₁-C₄)-alkyl, wobei jeder der letztgenannten 4 Reste am Ring unsubstituiert oder durch einen oder mehrere Reste aus der Gruppe Halogen, (C₁-C₄)-Alkyl, (C₁-C₄)-Alkoxy, (C₁-C₄)-Haloalkyl und (C₁-C₄)-Haloalkoxy substituiert ist und Heterocyclyl jeweils einen heterocyclischen Rest mit 1 bis 3 Heteroatomen aus der Gruppe N, O und S bedeutet, steht.

3. Verfahren nach Anspruch oder 2, **dadurch gekennzeichnet, dass** in der Formel (IV)
R¹ für Wasserstoff, (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH2)ₙCO₂R⁴, -COR⁴, -SOR⁴ oder -SO₂R⁴, wobei n eine ganze Zahl von 0 bis12 bedeutet, oder Phenyl, steht,
R² für Wasserstoff, (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl steht,
R³ für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl steht und
R⁴ für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl steht.

4. Verfahren nach einem der Ansprüche 1 bis 3, **dadurch gekennzeichnet, dass** für die thermische Decarboxylierung in Stufe (c) ein aromatisches oder heteroaromatisches tert. Amin, ein Phasen Transfer Katalysator und/oder eine Fluoridquelle verwendet wird.

5. Verfahren nach einem der Ansprüche 1 bis 4, **dadurch gekennzeichnet, dass** die Decarboxylierung in Stufe (c) bei Temperaturen zwischen 60 und 200 °C durchgeführt wird.

6. Verfahren nach einem der Ansprüche 1 bis 5, **dadurch gekennzeichnet, dass** die Umsetzungen unter Verwendung eines chiralen, nicht-racemischen Edukts durchgeführt und in der Stufe (c) die Decarboxylierung in Gegenwart eines Decarboxylierungsreagenzes oder-katalysators aus der Gruppe DMAP, KF, CsF, Tetraalkylammoniumchloride, Tetralkylphosphoniumchloride, Hexaalkylguanidiniumchloride, Hexaalkylguanidiniumfluoride und Mischungen der Verbindungen aus der vorgenannten Gruppe enantioselektiv geführt wird.

7. Verfahren nach einem der Ansprüche 1 bis 6, **dadurch gekennzeichnet, dass** das Lösungsmittel in Stufe (c) ein halogenierter Aromat, ein N,N-dialkyliertes Amid, Sulfolan oder ein Ester ist.

8. Verfahren nach einem der Ansprüche 1 bis 7, **dadurch gekennzeichnet, dass** in Stufe (c) 0,005 bis 6 Mol Decarboxylierungs-reagenz/-katalysators pro Mol Verbindung der Formel (III) eingesetzt wird.

9. Verfahren zur Herstellung von Verbindungen der Formel (IV), gegebenenfalls in optisch aktiver Form, 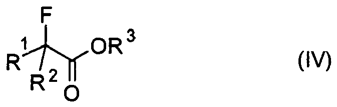 worin
R¹ für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R² für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder gegebenenfalls substituiertes Aryl, vorzugsweise Phenyl, das unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist, steht,
R³ für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R⁴ für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
**dadurch gekennzeichnet, dass** man
(c) eine Verbindung der Formel (III) 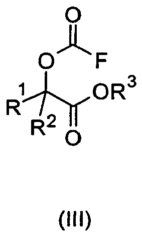 wobei in Formeln (III) R¹, R² und R³ wie in Formel (IV) definiert ist, thermisch, gegebenenfalls in Gegenwart eines Katalysators, unter Decarboxylierung zur Verbindung der genannten Formel (IV) umsetzt.

10. Verfahren zur Herstellung von Verbindungen der Formel (IV), gegebenenfalls in optisch aktiver Form, **dadurch gekennzeichnet, dass** man eine Verbindung der Formel (II), 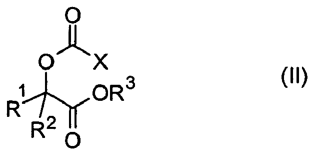
worin R¹, R² und R³ wie in Formel (IV) nach Anspruch 1 definiert sind und
X ein Halogenatom aus der Gruppe Cl oder Br bedeutet,
mit einem Fluorierungsreagenz zur Verbindung zur Verbindung der Formel (III) umsetzt 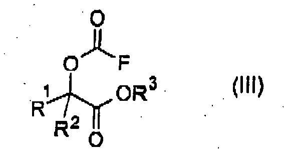 wobei in Formel (III) R¹, R² und R³ wie in der genannten Formel (IV) definiert sind,
und die erhaltenen Verbindung (III) thermisch, gegebenenfalls in Gegenwart eines Katalysators, unter Decarboxylierung zur Verbindung der genannten Formel (IV) umsetzt.

11. Verbindungen der Formel (III), gegebenenfalls in optisch aktiver Form, 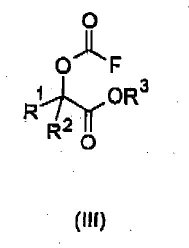 worin
R¹ für Wasserstoff, (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -CCR⁴, -SOR⁴ oder -SO₂R⁴, wobei n eine ganze Zahl von 0 bis 12 bedeutet, oder Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R² für (C₁-C₂₄)-Alkyl, (C₂-C₂₄)-Alkenyl, (C₂-C₂₄)-Alklnyl, (C₃-C₉)-Cycloalkyl, (C₁-C₆)-Alkyl-(C₃-C₉)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis 12 bedeutet, oder gegebenenfalls substituiertes Aryl, vorzugsweise Phenyl, das unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist, steht,
R³ für (C₁-C₂₄)-Alkyl, (C₂-C₂₄)-Alkenyl, (C₂-C₂₄)-Alkinyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht,
R⁴ für (C₁-C₂₄)-Alkyl. (C₂-C₂₄)-Alkenyl, (C₂-C₂₄)-Alklnyl, (C₃-C₉)-Cycloalkyl, (C₁-C₆)-Alkyl-(C₃-C₉)-cycloalkyl, Aryl, Heterocyclyl, Phenyl-(C₁-C₆)-alkyl oder Heterocyclyl-(C₁-C₆)-alkyl, wobei jeder der letztgenannten 4 Reste am Ring unsubstituiert oder durch einen oder mehrere Reste aus der Gruppe Halogen, (C₁-C₁₂)-Alkyl, (C₁-C₁₂)-Alkoxy, (C₁-C₁₂)-Haloalkyl und (C₁-C₁₂)-Haloalkoxy substituiert ist und Heterocyclyl jeweils einen heterocyclischen Rest mit 1 bis 3 Heteroatomen aus der Gruppe N, O und S bedeutet, steht.

12. Verbindungen der Formel (III) nach Anspruch 11, **dadurch gekennzeichnet, dass**
R¹ für Wasserstoff, (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder -SO₂R⁴, wobei n eine ganze Zahl von 0 bis12 bedeutet, oder Phenyl, steht,
R² für (C₁-C₆)-Alkyl, (C₂-C₆)-Alkenyl, (C₂-C₆)-Alkinyl, (C₃-C₆)-Cycloalkyl, (C₁-C₄)-Alkyl-(C₃-C₆)-cycloalkyl oder einen Rest der Formel -CO₂R⁴, -(CH₂)ₙCO₂R⁴, -COR⁴, -SOR⁴ oder SO₂R⁴, wobei n eine ganze Zahl von 0 bis12 bedeutet, oder Phenyl steht,
R³ für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl steht und
R⁴ für (C₁-C₆)-Alkyl, (C₂-C₆)-Alkenyl, (C₂-C₆)-Alkinyl, (C₃-C₆)-Cycloalkyl oder (C₁-C₄)-Alkyl-(C₃-C₆)-cycloalkyl steht,
vorzugsweise chirale, nicht-racemische Verbindungen der genannten Formel (III).

## Claims

1. A process for preparing compounds of the formula (IV), optionally in optically active form, 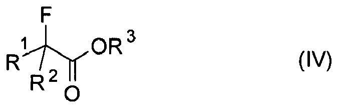 in which
R¹ is hydrogen, (C₁-C₂₄) -alkyl, (C₂-C₂₄) -alkenyl, (C₂-C₂₄)-alkynyl, (C₃-C₉)-cycloalkyl, (C₁-C₆) - alkyl- (C₃-C₉) -cycloalkyl or a radical of the formula -CO₂R⁴, - (CH₂) ₙCO₂R⁴, -COR⁴, -SOR⁴ or -SO₂R⁴, where n is an integer from 0 to 12, or aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂)-alkoxy, (C₁-C₁₂) -haloalkyl and (C₁-C₁₂) -haloalkoxy, and heterocyclyl is in each case a heterocyclic radical having 1 to 3 heteroatoms from the group of N, 0 and S,
R² is hydrogen, (C₁-C₂₄) -alkyl, (C₂-C₂₄) -alkenyl, (C₂-C₂₄)-alkynyl, (C₃-C₉)-cycloalkyl, (C₁-C₆)-alkyl-(C₃-C₉)-cycloalkyl or a radical of the formula -CO₂R⁴, - (CH₂)ₙCO₂R⁴, -COR⁴, -SOR⁴ or -SO₂R⁴, where n is an integer from 0 to 12, or optionally substituted aryl, preferably phenyl, which is unsubstituted or substituted by one or more radicals from the group of halogen, (C₁-C₁₂) -alkyl, (C₁-C₁₂) -alkoxy, (C₁-C₁₂) -haloalkyl and (C₁-C₁₂) -haloalkoxy,
R³ is (C₁-C₂₄) -alkyl, (C₂-C₂₄) -alkenyl, (C₂-C₂₄) alkynyl, (C₃-C₉) -cycloalkyl, (C₁-C₆) -alkyl-(C₃-C₉)-cycloalkyl, aryl, heterocyclyl, phenyl- (C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂) -alkoxy, (C₁-C₁₂)-haloalkyl and (C₁-C₁₂)-haloalkoxy, and heterocyclyl is in each case a heterocyclic radical having 1 to 3 heteroatoms from the group of N, 0 and S,
R⁴ is (C₁-C₂₄)-alkyl, (C₂-C₂₄) -alkenyl, (C₂-C₂₄) alkynyl, (C₃-C₉)-cycloalkyl, (C₁-C₆) -alkyl-(C₃-C₉)-cycloalkyl, aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂) -alkyl, (C₁-C₁₂) -alkoxy, (C₁-C₁₂)-haloalkyl and (C₁-C₁₂) -haloalkoxy, and heterocyclyl is in each case a heterocyclic radical having 1 to 3 heteroatoms from the group of N, O and S,
which comprises
(a) reacting a compound of the formula (I) 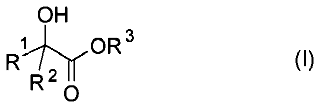 in which R¹, R² and R³ are each as defined in formula (IV)
with a dihalocarbonyl compound or an equivalent thereof to give the compound of the formula (II) [variant (a1)] or (III) [variant (a2)] 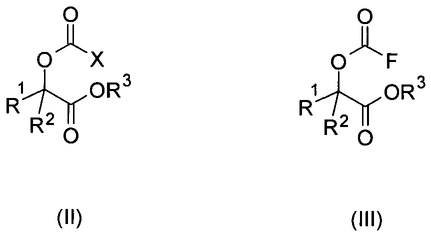 where, in the formulae (II) and (III),
R¹, R² and R³ are each as defined in formula (IV) and
X is a halogen atom from the group of Cl or Br,
and
(b) in the case that the compound (II) has been obtained in stage (a) according to variant (a1), reacting the compound (II) with a fluorinating reagent to give the compound of the formula (III) mentioned,
(c) reacting the compound of the formula (III) obtained in stage (a) or (b) thermally, optionally in the presence of a catalyst, with decarboxylation to give the compound of the formula (IV) mentioned.

2. The process as claimed in claim 1, wherein, in the formula (IV),
R¹ is hydrogen, (C₁-C₁₂) -alkyl, (C₂-C₁₂) -alkenyl, (C₂-C₁₂)-alkynyl, (C₃-C₆) -cycloalkyl, (C₁-C₄) - alkyl- (C₃-C₆) -cycloalkyl or a radical of the formula -CO₂R⁴, - (CH₂)ₙCO₂R⁴, -COR⁴, -SOR⁴ or -SO₂R⁴, where n is an integer from 0 to 12, or phenyl, heterocyclyl having from 3 to 6 ring atoms, phenyl-(C₁-C₄)-alkyl or heterocyclyl-(C₁-C₄)-alkyl, where each of the 4 latter radicals, on the ring, is unsubstituted or substituted by one or more radicals from the group of halogen, (C₁-C₄) -alkyl, (C₁-C₄)-alkoxy, (C₁-C₄) -haloalkyl and (C₁-C₄)-haloalkoxy, and heterocyclyl is in each case a heterocyclic radical having 1 to 3 heteroatoms from the group of N, O and S,
R² is hydrogen, (C₁-C₁₂) -alkyl, (C₂-C₁₂) -alkenyl, (C₂-C₁₂)-alkynyl, (C₃-C₆) -cycloalkyl, (C₁-C₄) - alkyl-(C₃-C₆)-cycloalkyl or a radical of the formula -CO₂R⁴, - (CH₂)ₙCO₂R⁴, -COR⁴, -SOR⁴ or -SO₂R⁴, where n is an integer from 0 to 12, or optionally substituted aryl, preferably phenyl, which is unsubstituted or substituted by one or more radicals from the group of halogen, (C₁-C₆) -alkyl, (C₁-C₆) -alkoxy, (C₁-C₆)-haloalkyl and (C₁-C₆)-haloalkoxy,
R³ is (C₁-C₁₂)-alkyl, (C₂-C₁₂) -alkenyl, (C₂-C₁₂) - alkynyl, (C₃-C₆)-cycloalkyl, (C₁-C₄)-alkyl-(C₃-C₆)-cycloalkyl, phenyl, heterocyclyl, phenyl- (C₁-C₄)-alkyl or heterocyclyl-(C₁-C₄) - alkyl, where each of the 4 latter radicals, on the ring, is unsubstituted or substituted by one or more radicals from the group of halogen, (C₁-C₄) -alkyl, (C₁-C₄) -alkoxy, (C₁-C₄)-haloalkyl and (C₁-C₄)-haloalkoxy, and heterocyclyl is in each case a heterocyclic radical having 1 to 3 heteroatoms from the group of N, 0 and S, and
R⁴ is (C₁-C₁₂) -alkyl, (C₂-C₁₂) -alkenyl, (C₂-C₁₂)-alkynyl, (C₃-C₆) -cycloalkyl, (C₁-C₄) -alkyl-(C₃-C₆)-cycloalkyl, phenyl, heterocyclyl, phenyl- (C₁-C₄)-alkyl or heterocyclyl-(C₁-C₄)-alkyl, where each of the 4 latter radicals, on the ring, is unsubstituted or substituted by one or more radicals from the group of halogen, (C₁-C₄) -alkyl, (C₁-C₄) -alkoxy, (C₁-C₄)-haloalkyl and (C₁-C₄)-haloalkoxy, and heterocyclyl is in each case a heterocyclic radical having 1 to 3 heteroatoms from the group of N, 0 and S.

3. The process as claimed in claim 1 or 2, wherein, in the formula (IV),
R¹ is hydrogen, (C₁-C₆)-alkyl, (C₂-C₆) -alkenyl, (C₂-C₆) -alkynyl, (C₃-C₆) -cycloalkyl, (C₁-C₄)-alkyl- (C₃-C₆) -cycloalkyl or a radical of the formula -CO₂R⁴, - (CH₂)ₙCO₂R⁴, -COR⁴, -SOR⁴ or -SO₂R⁴, where n is an integer from 0 to 12, or phenyl,
R² is hydrogen, (C₁-C₆)-alkyl, (C₂-C₆) -alkenyl, (C₂-C₆) -alkynyl, (C₃-C₆) -cycloalkyl, (C₁-C₄)-alkyl- (C₃-C₆) -cycloalkyl or a radical of the formula -CO₂R⁴, - (CH₂)ₙCO₂R⁴, -COR⁴, -SOR⁴ or -SO₂R⁴, where n is an integer from 0 to 12, or phenyl,
R³ is (C₁-C₆)-alkyl, (C₂-C₆) -alkenyl, (C₂-C₆) -alkynyl, (C₃-C₆) -cycloalkyl or (C₁-C₄) -alkyl- (C₃-C₆) cycloalkyl and
R⁴ is (C₁-C₆)-alkyl, (C₂-C₆) -alkenyl, (C₂-C₆) -alkynyl, (C₃-C₆) -cycloalkyl or (C₁-C₄)-alkyl-(C₃-C₆)-cycloalkyl.

4. The process as claimed in one of claims 1 to 3, wherein an aromatic or heteroaromatic tertiary amine, a phase transfer catalyst and/or a fluoride source is used for the thermal decarboxylation in stage (c).

5. The process as claimed in one of claims 1 to 4, wherein the decarboxylation in stage (c) is performed at temperatures between 60 and 200°C.

6. The process as claimed in one of claims 1 to 5, wherein the reactions are performed using a chiral, non-racemic reactant, and the decarboxylation in stage (c) is conducted enantioselectively in the presence of a decarboxylation reagent or catalyst from the group of DMAP, KF, CsF, tetraalkylammonium chlorides, tetralkylphosphonium chlorides, hexaalkylguanidinium chlorides, hexaalkylguanidinium fluorides and mixtures of the compounds from the aforementioned group.

7. The process as claimed in one of claims 1 to 6, wherein the solvent in stage (c) is a halogenated aromatic, an N,N-dialkylated amide, sulfolane or an ester.

8. The process as claimed in one of claims 1 to 7, wherein from 0.005 to 6 mol of decarboxylation reagent/catalyst are used in stage (c) per mole of compound of the formula (III).

9. A process for preparing compounds of the formula (IV), optionally in optically active form, 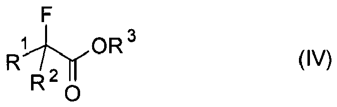 in which
R¹ is hydrogen, (C₁-C₂₄) -alkyl, (C₂-C₂₄)-alkenyl, (C₂-C₂₄)-alkynyl, (C₃-C₉)-cycloalkyl, (C₁-C₆)-alkyl- (C₃-C₉) -cycloalkyl or a radical of the formula -CO₂R⁴, -(CH₂)ₙCO₂R⁴, -COR⁴, -SOR⁴ or -SO₂R⁴, where n is an integer from 0 to 12, or aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂)-alkoxy, (C₁-C₁₂) -haloalkyl and (C₁-C₁₂)-haloalkoxy, and heterocyclyl is in each case a heterocyclic radical having 1 to 3 heteroatoms from the group of N, 0 and S,
R² is hydrogen, (C₁-C₂₄) -alkyl, (C₂-C₂₄)-alkenyl, (C₂-C₂₄)-alkynyl, (C₃-C₉) -cycloalkyl, (C₁-C₆)-alkyl-(C₃-C₉)-cycloalkyl or a radical of the formula -CO₂R⁴, -(CH₂)ₙCO₂R⁴, -COR⁴, -SOR⁴ or -SO₂R⁴, where n is an integer from 0 to 12, or optionally substituted aryl, preferably phenyl, which is unsubstituted or substituted by one or more radicals from the group of halogen, (C₁-C₁₂) -alkyl, (C₁-C₁₂) -alkoxy, (C₁-C₁₂) -haloalkyl and (C₁-C₁₂) -haloalkoxy,
R³ is (C₁-C₂₄)-alkyl, (C₂-C₂₄) -alkenyl, (C₂-C₂₄)-alkynyl, (C₃-C₉)-cycloalkyl, (C₁-C₆)-alkyl-(C₃-C₉)-cycloalkyl, aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂) -alkyl, (C₁-C₁₂)-alkoxy, (C₁-C₁₂) -haloalkyl and (C₁-C₁₂) -haloalkoxy, and heterocyclyl is in each case a heterocyclic radical having 1 to 3 heteroatoms from the group of N, 0 and S,
R⁴ is (C₁-C₂₄)-alkyl, (C₂-C₂₄)-alkenyl, (C₂-C₂₄) alkynyl, (C₃-C₉) -cycloalkyl, (C₁-C₆)-alkyl-(C₃-C₉)-cycloalkyl, aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂) -alkyl, (C₁-C₁₂) -alkoxy, (C₁-C₁₂)-haloalkyl and (C₁-C₁₂)-haloalkoxy, and heterocyclyl is in each case a heterocyclic radical having 1 to 3 heteroatoms from the group of N, 0 and S,
which comprises
(c) reacting a compound of the formula (III) 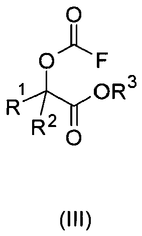 where, in formula (III), R¹, R² and R³ are each as defined in formula (IV), thermally, optionally in the presence of a catalyst, with decarboxylation to give the compound of the formula (IV) mentioned.

10. A process for preparing compounds of the formula (IV), optionally in optically active form, which comprises reacting a compound of the formula (II) 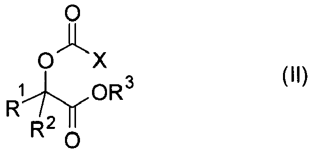 in which R¹, R² and R³ are each as defined in formula (IV) according to claim 1 and X is an halogen atom from the group of Cl and Br,
with a fluorinating reagent to give the compound of the formula (III) 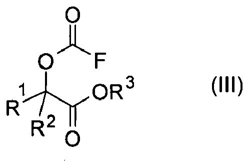 where, in formula (III), R¹, R² and R³ are each as defined in the formula (IV) mentioned,
and reacting the resulting compound (III) thermally, optionally in the presence of a catalyst, with decarboxylation to give the compound of the formula (IV) mentioned.

11. A compound of the formula (III), optionally in optically active form, 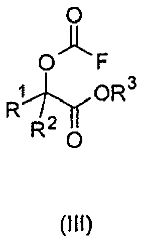 in which
R¹ is hydrogen, (C₁-C₂₄) -alkyl, (C₂-C₂₄) -alkenyl, (C₂-C₂₄)-alkynyl, (C₃-C₉)-cycloalkyl, (C₁-C₆)-alkyl-(C₃-C₉)-cycloalkyl or a radical of the formula -CO₂R⁴, - (CH₂)ₙCO₂R⁴, -COR⁴, -SOR⁴ or -SO₂R⁴, where n is an integer from 0 to 12, or aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂)-alkoxy, (C₁-C₁₂) -haloalkyl and (C₁-C₁₂)-haloalkoxy, and heterocyclyl is in each case a heterocyclic radical having 1 to 3 heteroatoms from the group of N, O and S,
R² is (C₁-C₂₄)-alkyl, (C₂-C₂₄)-alkenyl, (C₂-C₂₄)-alkynyl, (C₃-C₉)-cycloalkyl, (C₁-C₆)-alkyl-(C₃-C₉)-cycloalkyl or a radical of the formula -CO₂R⁴, - (CH₂)ₙCO₂R⁴, -COR⁴, -SOR⁴ or -SO₂R⁴, where n is an integer from 0 to 12, or optionally substituted aryl, preferably phenyl, which is unsubstituted or substituted by one or more radicals from the group of halogen, (C₁-C₁₂) -alkyl, (C₁-C₁₂)-alkoxy, (C₁-C₁₂)-haloalkyl and (C₁-C₁₂) -haloalkoxy,
R³ is (C₁-C₂₄)-alkyl, (C₂-C₂₄) -alkenyl, (C₂-C₂₄)-alkynyl, (C₃-C₉)-cycloalkyl, (C₁-C₆)-alkyl-(C₃-C₉)-cycloalkyl, aryl, heterocyclyl, phenyl-(C₁-C₆) -alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂)-alkoxy, (C₁-C₁₂)-haloalkyl and (C₁-C₁₂)-haloalkoxy, and heterocyclyl is in each case a heterocyclic radical having 1 to 3 heteroatoms from the group of N, O and S,
R⁴ is (C₁-C₂₄) -alkyl, (C₂-C₂₄) -alkenyl, (C₂-C₂₄)-alkynyl, (C₃-C₉) -cycloalkyl, (C₁-C₆) -alkyl-(C₃-C₉)-cycloalkyl, aryl, heterocyclyl, phenyl-(C₁-C₆)-alkyl or heterocyclyl-(C₁-C₆)-alkyl, where each of the 4 latter radicals, on the ring, is unsubstituted or substituted by one or more radicals from the group of halogen, (C₁-C₁₂)-alkyl, (C₁-C₁₂) -alkoxy, (C₁-C₁₂)-haloalkyl and (C₁-C₁₂)-haloalkoxy, and heterocyclyl is in each case a heterocyclic radical having 1 to 3 heteroatoms from the group of N, O and S.

12. A compound of the formula (III) as claimed in claim 11, wherein
R¹ is hydrogen, (C₁-C₆)-alkyl, (C₂-C₆) -alkenyl, (C₂-C₆) -alkynyl, (C₃-C₆) -cycloalkyl, (C₁-C₄)-alkyl-(C₃-C₆)-cycloalkyl or a radical of the formula -CO₂R⁴, -(CH₂)ₙCO₂R⁴, -COR⁴, -SOR⁴ or -SO₂R⁴, where n is an integer from 0 to 12, or phenyl,
R² is (C₁-C₆)-alkyl, (C₂-C₆) -alkenyl, (C₂-C₆)-alkynyl, (C₃-C₆) -cycloalkyl, (C₁-C₄) -alkyl- (C₃-C₆)-cycloalkyl or a radical of the formula -CO₂R⁴, -(CH₂)ₙCO₂R⁴, -COR⁴, -SOR⁴ or -SO₂R⁴, where n is an integer from 0 to 12, or phenyl,
R³ is (C₁-C₆)-alkyl, (C₂-C₆) -alkenyl, (C₂-C₆) -alkynyl, (C₃-C₆)-cycloalkyl or (C₁-C₄) -alkyl-(C₃-C₆)-cycloalkyl and
R⁴ is (C₁-C₆) -alkyl, (C₂-C₆) -alkenyl, (C₂-C₆) -alkynyl, (C₃-C₆) -cycloalkyl or (C₁-C₄) -alkyl-(C₃-C₆)-cycloalkyl,
preferably a chiral, non-racemic compound of the formula (III) mentioned.

## Revendications

1. Procédé pour la préparation de composés de formule (IV), éventuellement sous forme optiquement active, 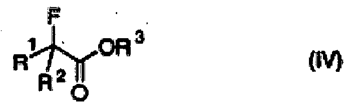 dans laquelle
R¹ représente un atome d'hydrogène, un groupe alkyle en C₁-C₂₄, alcényle en C₂-C₂₄, alcynyle en C₂-C₂₄, cycloalkyle en C₃-C₉, alkyl (C₁-C₆)-cycloalkyle(C₃-C₉) ou un radical de formule -CO₂R⁴, - (CH₂) ₙCO₂R⁴, -COR⁴, -SOR⁴ ou -SO₂R⁴, n représentant un nombre entier valant de 0 à 12, ou aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R² représente un atome d'hydrogène, un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉) ou un radical de formule -CO₂R⁴, - (CH₂)ₙCO₂R⁴, -COR⁴, -SOR⁴ ou -SO₂R⁴, n représentant un nombre entier valant de 0 à 12, ou un groupe aryle éventuellement substitué, de préférence phényle qui est non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂),
R³ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R⁴ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, O et S,
**caractérisé en ce que**
(a) on fait réagir un composé de formule (I) 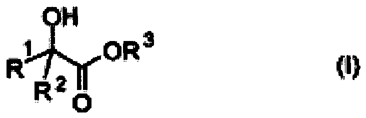 dans laquelle R¹, R² et R³ sont tels que définis dans la formule (IV),
avec un composé de type dihalogénocarbonyle ou un équivalent de celui-ci, pour aboutir au composé de formule (II) [variante (a1)] ou (III)
{variante (a2)], 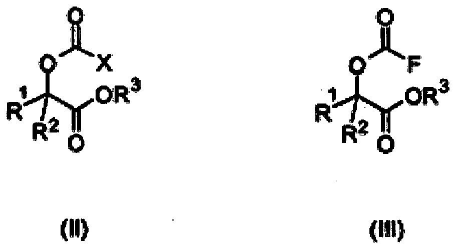 dans les formules (II) et (III)
R¹, R² et R³ étant tels que définis dans la formule (IV) et
X représentant un atome d'halogène choisi dans le groupe constitué par Cl et Br,
et
(b) dans le cas où dans l'étape (a) le composé (II) a été obtenu selon la variante (a1), on fait réagir le composé (II) avec un réactif de fluoration pour aboutir au composé de ladite formule (III),
(c) on convertit thermiquement, éventuellement en présence d'un catalyseur, avec décarboxylation, le composé de formule (III) obtenu après l'étape (a) ou (b), en le composé de ladite formule (IV).

2. Procédé selon la revendication 1, **caractérisé en ce que** dans la formule (IV)
R¹ représente un atome d'hydrogène, un groupe alkyle en C₁-C₁₂, alcényle en C₂-C₁₂, alcynyle en C₂-C₁₂, cycloalkyle en C₃-C₆, alkyl(C₁-C₄) - cycloalkyle(C₃-C₆) ou un radical de formule -CO₂R⁴, -(CH₂)ₙCO₂R⁴, -COR⁴, -SOR⁴ ou -SO₂R⁴, n représentant un nombre entier valant de 0 à 12, ou phényle, hétérocyclyle ayant de 3 à 6 atomes formant le cycle, phényl-alkyle(C₁-C₄) ou hétérocyclyl-alkyle(C₁-C₄), chacun des 4 radicaux nommés en dernier étant sur le cycle non substitué ou substitué par un ou plusieurs radicaux choisis dans le groupe constitué par halogéno, alkyle en C₁-C₄, alcoxy en C₁-C₄, halogénoalkyle(C₁-C₄) et halogénoalcoxy(C₁-C₄) et hétérocyclyle signifiant chaque fois un radical hétérocyclique comportant de 1 à 3 hétéroatomes choisis dans le groupe constitué par N, 0 et S,
R² représente un atome d'hydrogène, un groupe alkyle en C₁-C₁₂, alcényle en C₂-C₁₂, alcynyle en C₂-C₁₂, cycloalkyle en C₃-C₆, alkyl (C₁-C₄)-cycloalkyle(C₃-C₆) ou un radical de formule -CO₂R⁴, -(CH2)ₙCO₂R⁴, -COR⁴, -SOR⁴ ou -SO₂R⁴, n représentant un nombre entier valant de 0 à 12, ou un groupe aryle éventuellement substitué, de préférence phényle qui est non substitué ou substitué par un ou plusieurs radicaux choisis dans le groupe constitué par halogéno, alkyle en C₁-C₆, alcoxy en C₁-C₆, halogénoalkyle(C₁-C₆) et halogéno-alcoxy (C₁-C₆),
R³ représente un groupe alkyle en C₁-C₁₂, alcényle en C₂-C₁₂, alcynyle en C₂-C₁₂, cycloalkyle en C₃-C₆, alkyl(C₁-C₄)-cycloalkyle(C₃-C₆), phényle, hétérocyclyle, phényl-alkyle(C₁-C₄) ou hétérocyclyl-alkyle(C₁-C₄), chacun des 4 radicaux nommés en dernier étant sur le cycle non substitué ou substitué par un ou plusieurs radicaux choisis dans le groupe constitué par halogéno, alkyle en C₁-C₄, alcoxy en C₁-C₄, halogénoalkyle(C₁-C₄) et halogénoalcoxy(C₁-C₄) et hétérocyclyle signifiant chaque fois un radical hétérocyclique comportant de 1 à 3 hétéroatomes choisis dans le groupe constitué par N, 0 et S, et
R⁴ représente un groupe alkyle en C₁-C₁₂, alcényle en C₂-C₁₂, alcynyle en C₂-C₁₂, cycloalkyle en C₃-C₆, alkyl(C₁-C₄)-cycloalkyle(C₃-C₆), phényle, hétérocyclyle, phényl-alkyle(C₁-C₄) ou hétérocyclyl-alkyle(C₁-C₄), chacun des 4 radicaux nommés en dernier étant sur le cycle non substitué ou substitué par un ou plusieurs radicaux choisis dans le groupe constitué par halogéno, alkyle en C₁-C₄, alcoxy en C₁-C₄, halogénoalkyle(C₁-C₄) et halogénoalcoxy(C₁-C₄) et hétérocyclyle signifiant chaque fois un radical hétérocyclique comportant de 1 à 3 hétéroatomes choisis dans le groupe constitué par N, 0 et S.

3. Procédé selon la revendication 1 ou 2, **caractérisé en ce que** dans la formule (IV)
R¹ représente un atome d'hydrogène, un groupe alkyle en C₁-C₆, alcényle en C₂-C₆, alcynyle en C₂-C₆, cycloalkyle en C₃-C₆, alkyl(C₁-C₄) - cycloalkyle(C₃-C₆) ou un radical de formule -CO₂R⁴, - (CH₂)ₙCO₂R⁴, -COR⁴, -SOR⁴ ou -SO₂R⁴, n représentant un nombre entier valant de 0 à 12, ou phényle,
R² représente un atome d'hydrogène, un groupe alkyle en C₁-C₆, alcényle en C₂-C₆, alcynyle en C₂-C₆, cycloalkyle en C₃-C₆, alkyl(C₁-C₄)-cycloalkyle(C₃-C₆) ou un radical de formule -CO₂R⁴, -(CH2)ₙCO₂R⁴, -COR⁴, -SOR⁴ ou -SO₂R⁴, n représentant un nombre entier valant de 0 à 12, ou phényle,
R³ représente un groupe alkyle en C₁-C₆, alcényle en C₂-C₆, alcynyle en C₂-C₆, cycloalkyle en C₃-C₆ ou alkyl(C₁-C₄)-cycloalkyle(C₃-C₆) et
R⁴ représente un groupe alkyle en C₁-C₆, alcényle en C₂-C₆, alcynyle en C₂-C₆, cycloalkyle en C₃-C₆ ou alkyl(C₁-C₄)-cycloalkyle(C₃-C₆).

4. Procédé selon l'une quelconque des revendications 1 à 3, **caractérisé en ce que** pour la décarboxylation thermique dans l'étape (c) on utilise une amine tertiaire aromatique ou hétéroaromatique, un catalyseur de transfert de phase et/ou une source de fluorure.

5. Procédé selon l'une quelconque des revendications 1 à 4, **caractérisé en ce que** dans l'étape (c) on effectue la décarboxylation à des températures comprises entre 60 et 200 °C.

6. Procédé selon l'une quelconque des revendications 1 à 5, **caractérisé en ce qu'**on effectue en mode énantiosélectif les réactions en utilisant un produit de départ chiral, non racémique et dans l'étape (c) la décarboxylation en présence d'un catalyseur ou réactif de décarboxylation choisi dans le groupe constitué par la DMAP, KF, CsF, les chlorures de tétraalkylammonium, chlorures de tétraalkylphosphonium, chlorures d'hexaalkylguanidinium, fluorures d'hexa-alkylguanidinium et des mélanges des composés du groupe précité.

7. Procédé selon l'une quelconque des revendications 1 à 6, **caractérisé en ce que** le solvant dans l'étape (c) est un composé aromatique halogéné, un amide N,N-dialkylé, le sulfolane ou un ester.

8. Procédé selon l'une quelconque des revendications 1 à 7, **caractérisé en ce que** dans l'étape (c) on utilise 0,005 à 6 moles de catalyseur/ réactif de décarboxylation par mole de composé de formule (III).

9. Procédé pour la préparation de composés de formule (IV), éventuellement sous forme optiquement active, 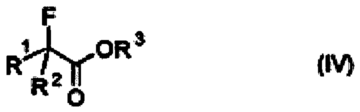 dans laquelle
R¹ représente un atome d'hydrogène, un groupe alkyle en C₁-C₂₄, alcényle en C₂-C₂₄, alcynyle en C₂-C₂₄, cycloalkyle en C₃-C₉, alkyl (C₁-C₆)-cycloalkyle(C₃-C₉) ou un radical de formule -CO₂R⁴, -(CH₂)ₙCO₂R⁴, -COR⁴, -SOR⁴ ou -SO₂R⁴, n représentant un nombre entier valant de 0 à 12, ou aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R² représente un atome d'hydrogène, un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉) ou un radical de formule -CO₂R⁴, -(CH₂)ₙCO₂R⁴, -COR⁴, -SOR⁴ ou -SO₂R⁴, n représentant un nombre entier valant de 0 à 12, ou un groupe aryle éventuellement substitué, de préférence phényle qui est non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogéno-alcoxy(C₁-C₁₂) ,
R³ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R⁴ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
**caractérisé en ce que**
(c) on convertit thermiquement, éventuellement en présence d'un catalyseur, avec décarboxylation, un composé de formule (III) 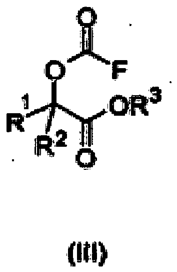 dans la formule (III) R¹, R² et R³ étant tels que définis dans la formule (IV), en le composé de ladite formule (IV).

10. Procédé pour la préparation de composés de formule (IV), éventuellement sous forme optiquement active, **caractérisé en ce qu'**on fait réagir un composé de formule (II), 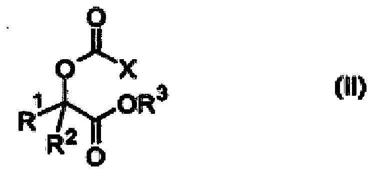 dans laquelle R¹, R² et R³ sont tels que définis dans la formule (IV) selon la revendication 1 et X représente un atome d'halogène choisi dans le groupe constitué par Cl et Br,
avec un réactif de fluoration, pour aboutir au composé de formule (III) 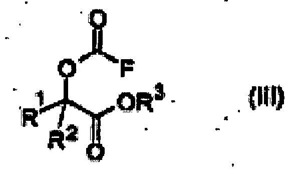 dans la formule (III) R¹, R² et R³ étant tels que définis dans ladite formule (IV),
et on convertit thermiquement le composé (III) obtenu, éventuellement en présence d'un catalyseur, avec décarboxylation, en le composé de ladite formule (IV).

11. Composés de formule (III), éventuellement sous forme optiquement active, 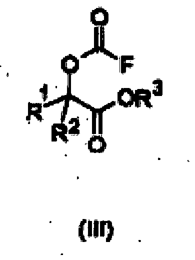 dans laquelle
R¹ représente un atome d'hydrogène, un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉) ou un radical de formule -CO₂R⁴, -(CH₂)ₙCO₂R⁴, -COR⁴, -SOR⁴ ou -SO₂R⁴, n représentant un nombre entier valant de 0 à 12, ou aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R² représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉) ou un radical de formule -CO₂R⁴, - (CH₂)ₙCO₂R⁴, -COR⁴, -SOR⁴ ou -SO₂R⁴, n représentant un nombre entier valant de 0 à 12, ou un groupe aryle éventuellement substitué, de préférence phényle qui est non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂),
R³ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, 0 et S,
R⁴ représente un groupe alkyle en C₁-C₂₄, alcényle en C₂-C₂₄, alcynyle en C₂-C₂₄, cycloalkyle en C₃-C₉, alkyl(C₁-C₆)-cycloalkyle(C₃-C₉), aryle, hétérocyclyle, phényl-alkyle(C₁-C₆) ou hétérocyclyl-alkyle(C₁-C₆), chacun des 4 radicaux nommés en dernier étant sur le cycle non substitué ou substitué par un ou plusieurs radicaux choisis dans le groupe constitué par halogéno, alkyle en C₁-C₁₂, alcoxy en C₁-C₁₂, halogénoalkyle(C₁-C₁₂) et halogénoalcoxy(C₁-C₁₂) et hétérocyclyle signifiant chaque fois un radical hétérocyclique comportant de 1 à 3 hétéroatomes choisis dans le groupe constitué par N, O et S.

12. Composés de formule (III) selon la revendication 11, **caractérisés en ce que**
R¹ représente un atome d'hydrogène, un groupe alkyle en C₁-C₆, alcényle en C₂-C₆, alcynyle en C₂-C₆, cycloalkyle en C₃-C₆, alkyl(C₁-C₄)-cycloalkyle(C₃-C₆) ou un radical de formule -CO₂R⁴, - (CH₂)ₙCO₂R⁴, -COR⁴, -SOR⁴ ou -SO₂R⁴, n représentant un nombre entier valant de 0 à 12, ou phényle,
R² représente un groupe alkyle en C₁-C₆, alcényle en C₂-C₆, alcynyle en C₂-C₆, cycloalkyle en C₃-C₆, alkyl(C₁-C₄)-cycloalkyle(C₃-C₆) ou un radical de formule -CO₂R⁴, -(CH₂)ₙCO₂R⁴, -COR⁴, -SOR⁴ ou -SO₂R⁴, n représentant un nombre entier valant de 0 à 12, ou phényle,
R³ représente un groupe alkyle en C₁-C₆, alcényle en C₂-C₆, alcynyle en C₂-C₆, cycloalkyle en C₃-C₆ ou alkyl(C₁-C₄)-cycloalkyle (C₃-C₆) et
R⁴ représente un groupe alkyle en C₁-C₆, alcényle en C₂-C₆, alcynyle en C₂-C₆, cycloalkyle en C₃-C₆ ou alkyl(C₁-C₄)-cycloalkyle(C₃-C₆).
de préférence composés de ladite formule (III) chiraux, non racémiques.
